(19) Europäisches Patentamt
European Patent Office
Office européen des brevets

(11) Publication number: **0 302 715 B1**

(12) **EUROPEAN PATENT SPECIFICATION**

(45) Date of publication of patent specification: **22.04.92**

(51) Int. Cl.[5]: **G01N 33/566**, G01N 33/545, G01N 33/546, G01N 33/569, G01N 33/76, G01N 33/541

(21) Application number: **88307171.4**

(22) Date of filing: **03.08.88**

(54) **Avidin- and biotin-immobilized reagents, analytical elements and methods of use.**

(30) Priority: **03.08.87 US 81206**
**18.12.87 US 136165**

(43) Date of publication of application:
**08.02.89 Bulletin 89/06**

(45) Publication of the grant of the patent:
**22.04.92 Bulletin 92/17**

(84) Designated Contracting States:
**CH DE FR GB LI SE**

(56) References cited:
**US-A- 4 161 407**
**US-A- 4 486 530**
**US-A- 4 496 654**
**US-A- 4 548 870**
**US-A- 4 582 810**

(73) Proprietor: **EASTMAN KODAK COMPANY (a New Jersey corporation)**
**343 State Street**
**Rochester New York 14650(US)**

(72) Inventor: **Sutton, Richard Calvin, c/o Eastman Kodak Co.**
**Patent Dept., 343 State Street**
**Rochester, NY 14650(US)**
Inventor: **Danielson, Susan Jean, c/o Eastman Kodak Co.**
**Patent Dept., 343 State Street**
**Rochester, NY 14650(US)**
Inventor: **Burdick, Brent Arthur, c/o Eastman Kodak Co.**
**Patent Dept., 343 State Street**
**Rochester, NY 14650(US)**
Inventor: **Warren, Harold Chester, III, c/o Eastman Kodak Co.**
**Patent Dept., 343 State Street**
**Rochester, NY 14650(US)**
Inventor: **Snyder, Brian Anthony, c/o Eastman Kodak Co.**
**Patent Dept., 343 State Street**
**Rochester, NY 14650(US)**
Inventor: **McClune, Gregory Joseph, c/o Eastman Kodak Co.**
**Patent Dept., 343 State Street**
**Rochester, NY 14650(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**EP 0 302 715 B1**

Inventor: **Wu, Annie Liu, c/o Eastman Kodak Co.**
**Patent Dept., 343 State Street**
**Rochester, NY 14650(US)**

(74) Representative: **Phillips, Margaret Dawn et al**
**Kodak Limited Patent Department Headstone Drive**
**Harrow, Middlesex HA1 4TY(GB)**

## Description

The present invention relates to water-insoluble reagents which are useful in various analytical procedures. It also relates to analytical elements and methods using these reagents.

There is a continuous need in medical practice, research and diagnostic procedures for rapid, accurate, quantitative determinations of biological substances which are present in biological fluids at low concentrations. For example, the presence of drugs, narcotics, hormones, steroids, polypeptides, prostaglandins or infectious organisms in blood, urine, saliva, vaginal secretions, seminal fluids and other biological fluids has to be determined in an accurate and rapid fashion for suitable diagnosis or treatment.

To provide such determinations, various methods have been devised for isolating and identifying biological substances employing specific binding reactions between the substance to be detected and receptors reactive with that substance. Radioactive or enzyme labels have been used to detect the resulting reactive complex.

One particular type of test which has been developed is an agglutination test which is useful for the detection of antigens which have multiple sites for antibody reactivity. In such a test, antibody molecules can be bound in a suitable fashion to water-insoluble particles. Antibody-antigen reaction at multiple sites causes the particles to agglutinate and precipitate. Suitable separation and detection means have been devised to make the agglutinate readily observable, including for example, the use of particles containing a tracer material.

Another useful method for detecting biological substances in fluids is what is known in the art as a "sandwich" assay. Such an assay involves "sandwiching" the compound of interest (such as an antigen) with two or more receptor molecules (such as antibodies) which complex with the compound at different and noninterfering sites. In most sandwich assays, one or more of the receptor molecules are suitably immobilized on an insoluble carrier such as small particles, membranes, plates, test wells or similar objects.

Attachment of antibodies or receptor molecules to insoluble carrier materials has been achieved in the past in a number of ways. Early work relied on adsorption of the molecules, but it was realized that adsorption is generally not a strong method of attachment. Later researchers found that the molecules could be covalently attached by reaction of certain functional groups of the molecules with specially designed reactive groups on the carrier material. For example, proteins have been attached by reacting carboxy groups of particles or supports with an activating compound which renders the groups reactive with amino groups of a protein. Carbodiimides are examples of useful activating compounds.

Avidin is a protein found in egg whites. Biotin (hexahydro-2-oxo-1H-thieno[3,4]imidazole-4-pentanoic acid), also known as Vitamin H, is a relatively small water-soluble molecule. These materials are known to react specifically with each other to form a very strong and stable complex in which each of the four subunits of avidin binds a biotin molecule. This strong binding is maintained even when either biotin or avidin or both are bound covalently to other materials. The reaction has been used to enhance agglutination of erythrocytes and provided researchers with a means for various biochemical and diagnostic studies.

Competitive immunoassays are known in which antibodies conjugated to biotin are allowed to compete with the unknown analyte and a known quantity of enzyme-labeled analyte. The amount of antibody-analyte complexes is readily determined by insolubilizing the complexes by adding avidin attached to a carrier material. Avidin is bound to a solid support such as particles, filter paper, glass or plastic object by covalent attachment, for example covalent attachment to benzoquinone-activated sepharose.

U.S. Patent 4,582,810 describes the attachment of avidin to latex particles having free carboxyl groups on their surfaces. As described therein, the conventional procedure for covalently attaching avidin to the particles involves the use of a water-soluble carbodiimide in an activation step. While producing reagents, this procedure tends to activate the exposed reactive groups of the protein avidin as well as the carboxyl groups on the particles. The result is intramolecular and intermolecular crosslinking or polymerization of avidin, and a significant portion of the reagent is impaired from complexation with biotin. In addition, there may be premature agglutination of the insolubilized reagent due to the cross-reactivity of the activating compound. These problems present a serious economic loss as well as an impairment of diagnostic sensitivity. It has also been evident that carbodiimides provide a reactive intermediate for avidin attachment which is unstable and must be used immediately.

Various other reagents have been prepared with particles having reactive groups such as epoxides, aldehydes, amino groups and diazonium salts. All of these groups have disadvantages. Epoxide groups are not stable, so that the particles cannot be stored for very long. Particles having aldehyde groups generally tend to agglutinate prematurely. The aldehyde groups also prematurely oxidize, thereby losing binding activity. Particles with amine groups are like the carboxylated materials by requiring an additional activation step for attachment. Diazonium compounds are unstable and therefore undesirable to work with.

Hence, reagents which are composed of avidin or biotin covalently attached to a water-insoluble particle would be very useful in diagnostic methods. However, it would be desirable to have such reagents which are readily prepared in an efficient manner and under conditions which are not limiting and which do not reduce sensitivity or generate other undesirable results. It would be particularly desirable to avoid the use of conventional carbodiimide chemistry for attachment whereby premature crosslinking and agglutination are prominent. It would also be desirable to have reagents for insolubilizing immunological species without directly attaching the species to the insoluble carrier material. Yet the resulting attachment should be stronger than that achieved through mere adsorption.

The problems noted above with conventional reagents have been overcome with a water-insoluble reagent comprising:

a polymeric particle comprising a polymer on at least its outer surface which is derived from one or more ethylenically unsaturated polymerizable monomers,

the reagent characterized in that at least one of the monomers has reactive groups which are activated 2-substituted ethylsulfonyl, vinylsulfonyl or active halogen atom groups, and the particle is covalently attached through the reactive groups on the outer surface of the particle to avidin, biotin or an avidin or biotin derivative.

This invention also provides an element comprising an absorbent carrier material having one or more zones, and characterized wherein it contains in one or more of the zones the water-insoluble reagent described above.

Further, the present invention provides a method for the determination of a compound of biological interest in an aqueous liquid comprising:

A. contacting the liquid with the water-insoluble reagent described above,

B. forming a reaction product of the reagent with the biological compound, and

C. determining the amount of the biological compound as a result of the presence of the reaction product.

The present invention further provides a method for the determination of a compound of biological interest in an aqueous liquid comprising:

A. contacting the liquid with a water-insoluble reagent comprising:

a core/shell polymeric particle in which the shell comprises a polymer which is represented by the formula:

$$-(A)_x-(B)_y-(D)_z-$$

wherein -A- represents recurring units derived from one or more hydrophobic ethylenically unsaturated monomers,

-B- represents recurring units derived from one or more ethylenically unsaturated monomers having a reactive group which is an activated 2-substituted ethylsulfonyl, vinylsulfonyl or active halogen atom,

-D- represents recurring units derived from one or more ethylenically unsaturated monomers other than those represented by -A- or -B-,

x is from 0 to 99.9 mole percent, y is from 0.1 to 100 mole percent, and z is from 0 to 20 mole percent,

the particle being covalently attached through the reactive groups on the outer surface of the shell to avidin or a derivative thereof,

B. forming a reaction product of the reagent with a biotinylated reagent comprised of biotin or a derivative thereof attached to a first receptor molecule for the biological compound so as to form an insoluble specific binding complex between avidin and the biotinylated reagent,

C. prior to, simultaneously with or subsequent to contacting step A, contacting the biological compound with a second receptor molecule which is capable of participating in a specific binding reaction with the biological compound but which is not reactive with the first receptor molecule, the second receptor being labeled with a detectable tracer material,

so as to form a labeled insoluble complex, and

D. detecting the labeled insoluble complex.

The present invention provides stable reagents which can be used in a variety of analytical and diagnostic procedures to great advantage. The polymer particles used to prepare the reagents have readily available functional groups which readily react with avidin, biotin or avidin or biotin derivatives. The useful functional groups include an activated 2-substituted ethylsulfonyl group, vinylsulfonyl group or a group

containing an active halogen atom. The reaction between avidin or an avidin derivative with particles containing these groups can be accomplished without the need for activators such as carbodiimides and additional activation steps. Therefore, the problems associated with the activation of carboxy groups (that is premature crosslinking and agglutination) are generally avoided.

In addition, preferred reagents of this invention wherein the particles have reactive vinylsulfonyl and activated 2-substituted ethylsulfonyl groups exhibit further advantages in that they can be reacted under mild pH conditions and low temperatures. Hence, the conditions of attachment are not as critical and lower temperatures, shorter reaction times and flexible mixing conditions can be employed without sacrificing sensitivity. Yet the reagent of this invention can be used to strongly attach immunological or other specific binding compounds to an insoluble carrier material.

The present invention provides a reagent for use in analytical methods and elements whereby a detectable complex is obtained using the high specific binding affinity of avidin for biotin. The methods can quickly provide a determination so that the assay can be performed in a doctor's office or in a consumer's home to provide immediate results. The test can be used to detect the presence or absence of a compound of biological interest in an aqueous liquid, such as a biological fluid.

A compound of biological interest is defined herein as any biological or chemical compound which has one or more sites for complexing with a corresponding specific binding receptor molecule. In one embodiment, the compound of biological interest may be avidin or biotin which can then be detected with the reagent of this invention containing the corresponding receptor therefor. For example, to detect biotin in a fluid, a reagent having avidin molecules attached thereto is used.

Compounds of biological interest can also be defined as ligands which specifically complex with a corresponding receptor molecule which is not biotin or avidin. For example, the compound could be an immunological species which is (1) any substance which, when presented to an immunocompetent host, will result in the production of a specific antibody capable of binding with that substance, or (2) the antibody so produced, which compound participates in an antigen-antibody reaction. In such embodiments, avidin, biotin or an avidin or biotin derivative is suitably attached to the receptor molecule which reacts specifically with the biological compound.

Representative ligands detectable with the present invention include primary amines, amino acids, peptides, polypeptides, proteins, lipoproteins, carbohydrates, glycoproteins, drugs, haptens, enzymes, steroids, lipids, nucleic acids, hormones, vitamins, polysaccharides, glycolipids, alkaloids, organisms (bacteria, protozoa, fungi, viruses, rickettsia and the like) and components thereof, blood components, tissue and organ antigens and other materials known to one skilled in the art. In some instances, the ligand is an antibody which is directed against a drug, hormone, antibiotic or other compound having antigenic properties. Alternatively, the ligand can be an antigenic material (including mono- or multi-valent or multideterminant antigens). In still another embodiment, the immunological species is an antibody which is directed against another antibody (that is, an anti-antibody). Both monoclonal and polyclonal antibodies can be used, and they can be whole molecules or various fragments thereof.

The reagent of the present invention is prepared by covalently attaching avidin, biotin or a derivative of either to water-insoluble polymeric particles of specific composition. The attachment is achieved through amino or sulfhydryl groups of the avidin, or avidin or biotin derivative which are available for reaction directly with reactive groups on the outer surface of the particles. "Direct" attachment means that the avidin or avidin or biotin derivative molecule is directly reacted with the particle groups. Alternatively, the material can be chemically modified to provide reactive sites for attachment as long as such modification does not adversely affect the sites where avidin and biotin will complex with each other. For example, biotin cannot be attached directly to such particles, but suitable biotin derivatives having suitable reactive groups (such as succinimidooxycarbonyl, maleimidooxycarbonyl or N′-bromoacetylhydrazinocarbonyl) can be attached to particles pretreated with a protein such as casein to provide amine groups that react with the biotin derivative. Biotin derivatives having reactive amine or sulfhydryl groups can be directly attached to the particles. In addition, avidin, biotin, or a derivative of either can be "indirectly" attached through a linking moiety which can be a protein, peptide, polypeptide, diamine or dimercaptan.

Avidin and biotin derivatives which can be used to prepare the reagents of this invention include streptavidin, succinylated avidin, monomeric avidin, biocytin (that is, biotin-ϵ-N-lysine), biocytin hydrazide, amine or sulfhydryl derivatives of 2-iminobiotin and biotinyl-ϵ-aminocaproic acid hydrazide.

Biotin derivatives, such as biotin-N-hydroxysuccinimide ester, biotinyl-ϵ-aminocaproic acid N hydroxysuccinimide ester, sulfosuccinimidyl 6-(biotin amido)hexanoate, N-hydroxysuccinimide-iminobiotin, biotinbromoacetylhydrazide, p-diazobenzoyl biocytin and 3-(N-maleimidopropionyl)biocytin, can also be attached to linking proteins after such proteins have been suitably attached to the polymeric particles.

The particles used in preparing the present invention comprise one or more polymers each of which is

prepared from one or more ethylenically unsaturated polymerizable monomers which are described below in more detail. At least one of such monomers provides the desired reactive groups on at least the surface of the particles. In some embodiments, the particles are homogeneous, that is, they are composed of the same polymer throughout. In other embodiments, the particles can be composed of two or more polymers, for example as core/shell particles or as graft copolymers.

The polymeric particles are generally water insoluble latex particles having an average particle size greater than 0.01 micrometers. Preferably they have an average particle size in the range of from 0.01 to 5 micrometers.

As described above, the polymeric particles useful in the practice of this invention comprise at least one polymer derived from at least one $\alpha,\beta$-ethylenically unsaturated polymerizable monomer having one or more of the reactive groups selected from the group consisting of activated 2-substituted ethylsulfonyl, vinylsulfonyl or an active halogen atom.

Monomers having an active halogen atom include vinyl chloroacetate, vinyl bromoacetate, haloalkylated vinyl aromatics (for example, chloromethylstyrene or bromomethylstyrene), haloalkyl acrylic or methacrylic esters (for example, chloroethyl methacrylate, 3-chloro-2-hydroxypropyl methacrylate and 3-chloromethyl acrylate) and others known to one skilled in the art. The haloalkylated vinyl aromatics, for example those having active haloalkyl groups of 1 to 3 carbon atoms, are preferred when the active halogen atom is used as the reactive group. Chloromethylstyrene is most preferred.

As noted above, the monomers having active halogen atoms exhibit many advantages over the materials of the art. However, monomers having activated 2-substituted ethylsulfonyl and vinylsulfonyl groups possess additional advantages in that proteins can be attached to the polymers under milder conditions and require less process control during manufacture. This renders manufacture more efficient and less costly. A number of representative monomers having the requisite pendant groups are known in the art, including U.S. Patents 4,161,407 and 4,548,870.

Preferred activated 2-substituted ethylsulfonyl and vinylsulfonyl monomers can be represented by the formula (I):

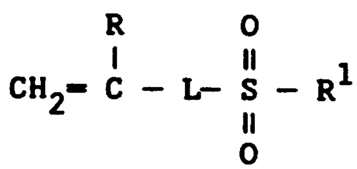 (I)

wherein R is hydrogen or substituted or unsubstituted alkyl (generally of 1 to 6 carbon atoms, such as methyl, ethyl, isopropyl or hexyl). Preferably, R is hydrogen or methyl.

$R^1$ is $-CH=CHR^2$ or $-CH_2CH_2X$ wherein X is a leaving group which is displaced by a nucleophile or is eliminated in the form of HX by treatment with a base (such as halo, acetoxy, alkylsulfonyloxy such as methylsulfonyloxy, arylsulfonyloxy such as p-tolylsulfonyloxy, trialkylammonio, for example, a trimethylammonio salt or pyridinio salt). $R^2$ is hydrogen, substituted or unsubstituted alkyl (generally of 1 to 6 carbon atoms as defined for R), or substituted or unsubstituted aryl (generally of 6 to 12 nuclear carbon atoms, such as phenyl, naphthyl, xylyl or tolyl). Preferably, $R^1$ is $-CH_2CH_2X$. This group, which is an activated 2-substituted ethyl group, can be substituted with any group which does not impair the displacement of the leaving group X.

L is a linking group which can be a substituted or unsubstituted alkylene generally having 1 to 20 carbon and hetero atoms in the backbone. This definition of alkylene is meant to include alkylene groups interrupted or terminated with oxy, thio, $-NR^3-$ [wherein $R^3$ is hydrogen, substituted or unsubstituted alkyl of 1 to 6 carbon atoms (such as methyl, chloromethyl or 2-hydroxyethyl) or substituted or unsubstituted aryl of 6 to 10 carbon atoms (such as phenyl, naphthyl or xylyl)], ester (-COO-), amide (-CONH-), urylene

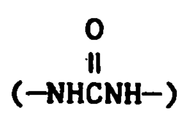,

sulfonyl ($-SO_2-$), carbonate, sulfonamide, azo, phosphono or other similar groups. Representative alkylene groups include methylene, ethylene, isobutylene, hexamethylene, carbonyloxyethyleneoxycarbonylethylene, methylenebis(iminocarbonyl)ethylene, carbonyloxydodecylenecarbonyloxyethylene, carbonyliminomethyleneiminocarbonyliminoethylene, carbonyliminomethyleneiminocarbonylethylene and other groups described or suggested by U.S. Patents 4,161,407 and 4,548,870.

L can also be substituted or unsubstituted arylene generally having 6 to 12 nuclear carbon atoms. Representative arylene groups include phenylene, tolylene, naphthylene and others noted in the patents mentioned above. Also included in this definition of L are divalent groups which are combinations of one or more of each of the alkylene and arylene groups defined above (for example, arylenealkylene, alkylenearylenealkylene and others readily determined by one of ordinary skill in the art), as well as such combinations which are interrupted or terminated by one or more amide or ester groups (for example, carbonyliminoarylenealkylene). Preferably, L is substituted or unsubstituted phenylenealkylene [for example, substituted with one or more alkyl groups (as defined for R), alkoxy groups (generally of 1 to 6 carbon atoms, for example, methoxy, propoxy or butoxy) or halo groups], carbonyliminoarylenealkylene (wherein arylene and alkylene are defined above), or carbonyliminoalkyleneiminocarbonylalkylene (wherein alkylene are defined above).

Representative useful monomers include m & p-(2-chloroethylsulfonylmethyl)styrene, m & p-[2-(p-tolylsulfonyloxy)ethylsulfonylmethyl]styrene, m & p-vinylsulfonylmethylstyrene, N-[m & p-(2-chloroethylsulfonylmethyl)phenyl]acrylamide, and N-[2-(2-chloroethylsulfonyl)ethylformamidomethyl]acrylamide. The first monomer is preferred.

One or more of the monomers described above can be polymerized individually or in combination to form homo- or copolymers. Alternatively, and preferably, one or more of them are copolymerized with at least one other ethylenically unsaturated polymerizable monomer. Generally such monomers provide various desirable properties such as hydrophobicity, dispersibility or other features. Preferred polymers can be represented by the formula (II):

$$-(A)_x-(B)_y-(D)_z- \qquad (II)$$

wherein -A- represents recurring units derived from one or more hydrophobic ethylenically unsaturated monomers,

-B- represents recurring units derived from one or more ethylenically unsaturated monomers having the requisite reactive groups described above, and

-D- represents recurring units derived from one or more ethylenically unsaturated monomers which are different than those represented by -A- or -B-.

In formula (II), x is from 0 to 99.9 mole Percent, y is from 0.1 to 100 mole percent, and z is from 0 to 20 mole percent. Preferably, x is from 45 to 99.5 mole percent, y is from 0.5 to 50 mole percent, and z is from 0 to 10 mole percent.

Monomers from which the -A- recurring units are derived, both in general and in preferred embodiments, are hydrophobic and form homopolymers that are insoluble in water. Preferably, these monomers have aromatic groups. Representative hydrophobic monomers include, but are not limited to, styrene and styrene derivatives (for example, 4-vinyltoluene, 2,5-dimethylstyrene, 4-t-butylstyrene, 2-chlorostyrene and others known in the art), acrylic and methacrylic acid esters and amides (for example, n-butyl acrylate, propyl methacrylate, methyl acrylate, methyl methacrylate, ethyl methacrylate, 2-ethylhexyl methacrylate, N-phenylacrylamide and others known in the art), acrylonitrile and vinyl acetate.

This polymer can be crosslinked, if desired, in any suitable fashion. One method is to incorporate a small amount, that is up to 10 mole percent, and preferably from 0.3 to 5 mole percent, of a monomer having two or more ethylenically unsaturated polymerizable groups. These monomers are included among the hydrophobic monomers from which A is derived. Representative monomers are described in Research Disclosure, publication 19551, July, 1980, page 304, and include for example, divinylbenzene, ethylene dimethacrylate, N,N′-methylenebisacrylamide, 2,2-dimethyl-1,3-propylene diacrylate, allyl acrylate, ethylidyne trimethacrylate and ethylene diacrylate.

Preferred monomers from which the -A- recurring units are derived are vinyl aromatic monomers, especially styrene and styrene derivatives.

The monomers from which the -B- recurring units are derived are those having the reactive groups described above.

Monomers from which the -D- recurring units are derived include monomers different than those from which -A- and -B- are derived. Specifically, the -D- recurring units are derived from monomers which impart aqueous dispersion stability to the particles or other properties. Representative monomers include, but are

not limited to, anionic monomers such as sodium 2-acrylamido-2-methylpropanesulfonate, acrylic acid, methacrylic acid, 2-carboxyethyl acrylate, styrene sulfonic acid, potassium salt and m & p-carboxymethylstyrene and other ethylenically unsaturated polymerizable sulfonates, carboxylates, sulfates and phosphonates, other hydrophilic but nonionic monomers, such as 2-hydroxyethyl acrylate and 2-hydroxyethyl methacrylate and others known to one skilled in the art.

Preferred monomers from which the -D- units are derived are acrylic acid, methacrylic acid, sodium 2-acrylamido-2-methylpropanesulfonate, m & p-carboxymethylstyrene and p-styrenesulfonic acid, potassium salt.

Representative polymers of the monomers described above include the following: poly(m & p-chloromethylstyrene), poly(styrene-co-m & p-chloromethylstyrene-co-2-hydroxyethyl acrylate) (67:30:3 molar ratio), poly(styrene-co-m & p-chloroethylsulfonylmethylstyrene) (95.5:4.5 molar ratio), poly{styrene-co-N-[m & p-(2-chloroethyl-sulfonylmethyl)phenyl]acrylamide} (99.3:0.7 molar ratio), poly(m & p-chloromethylstyrene-co-methacrylic acid)(95:5, 98:2 and 99.8:0.2 molar ratio), poly(styrene-co-m & p-chloroethylsulfonylmethylstyrene-co-methacrylic acid)(93.5:4.5:2 molar ratio), poly{styrene-co-N-[m & p-(2-chloroethylsulfonylmethyl)phenyl]acrylamide-co-methacrylic acid}(97.3:0.7:2 molar ratio), and poly(styrene-co-m & p-chloromethylstyrene)(70:30 molar ratio).

As noted above, the particles useful in the practice of this invention can be homogeneously composed of one of the polymers described above, or a mixture thereof. Alternatively, the polymers described above can be an outer graft or shell of a grafted copolymer or core shell particle, respectively.

The polymeric particles can be prepared using any suitable polymerization technique, including emulsion (including batch, semi-continuous and continuous) and suspension polymerization techniques, graft copolymerization, and others known to one skilled in the polymer chemistry art. Emulsion polymerization is preferred as it can be used to provide particles without the use of surfactants or emulsifiers as described for example in U.S. Patent 4,415,700 and Research Disclosure Publication 15963 (July, 1977). Other details of preparatory methods can be found in U.S. Patents 4,161,407 and 4,548,870.

Staged emulsion polymerization can be used to provide a core shell polymer composed of two different polymers. Emulsion polymerization of the core is carried to substantial completion by continuously adding reactants to a reaction vessel under standard conditions. Monomers and catalysts needed to make the shell polymer are then continuously added to the vessel containing the latex of the core polymer. In this manner, the shell has a definite known composition rather than being a mixture of core and shell monomers.

The general procedure for preparing the reagent of this invention includes covalently attaching avidin, biotin or an avidin or biotin derivative to the particles using generally known reactions. With the active halogen atom, 2-substituted activated ethylsulfonyl and vinylsulfonyl groups, avidin or an avidin derivative can be directly attached to the particles. Biotin or derivatives thereof can be attached indirectly as noted above. Generally, the polymer particles are mixed with the material to be attached in an aqueous buffered solution (pH generally from 7 to 10) and a concentration of from 0.01 to 40 weight percent polymer particles (preferably from 0.01 to 10 weight percent). The amount of avidin, biotin or a derivative of either is at a weight ratio to polymer of from 0.1:1000 to 1:10, and preferably from 1:100 to 1:10. Mixing is carried out at a temperature in the range of from 5 to 50 ° C, and preferably at from 5 to 40 ° C, for from 0.5 to 48 hours. Any suitable buffer can be used. The details of a representative preparatory procedure are illustrated in Example 1 below.

The procedural details of direct and indirect attachment of avidin, biotin or derivatives thereof are known in the art.

The polymeric particles used in this invention can have a detectable tracer material associated therewith if desired. A tracer is a material which is detectable with the unaided eye or with appropriate equipment and techniques. The tracer material can be inside or outside of the particles. Useful tracers include, but are not limited to, radioisotopes which emit gamma rays, fluorescent compounds or dyes, bioluminescent compounds, chemiluminescent compounds, chromogens such as dyes and dye-formers which absorb in the visible or ultraviolet region of the electromagnetic spectrum, and others known to one skilled in the art.

The reagent of the present invention can be used in the determination (qualitative or quantitative measurement) of a biological compound in aqueous liquids. This determination can be made by merely determining the presence or absence of the compound, or by quantitatively determining the amount of compound. In particular, the invention can be used to assay biological fluids of animals, humans or plants, but preferably of humans. Such fluids include, but are not limited to, whole blood, plasma, sera, lymph, bile, urine, spinal fluid, seminal fluid, lacrimal fluid, vaginal secretions, sputum, perspiration and the like as well as stool specimens. It is also possible to assay fluid preparations of human or animal tissue such as skeletal muscle, heart, kidney, lungs, brains, bone marrow, skin and the like.

The present invention can be used to determine avidin, biotin or a derivative of either which is reactive

with the corresponding moiety which is a part of the reagent of this invention. For example, the reagent can be comprised of avidin attached to a particle, and the compound to be determined is biotin or a derivative thereof with which the avidin is reactive.

Alternatively, the biological compound is a ligand other than avidin or biotin, such as an antibody or antigen, which has one or more sites for complexation with one or more receptor molecules. Such receptor molecules are usually immunologically reactive species. At least one of the receptor molecules is conjugated with avidin or biotin. The ligand can be complexed with the receptors, and the entire complex can be insolubilized by reaction of the conjugated avidin or biotin with the reagent of this invention. For example, the ligand can be Streptococcus A antigen, an antigen from chlamydial or gonococcal organisms, HTLV antigens or antibodies (for example, HTLV-I or HTLV-II), HIV antigens or antibodies (for example, HIV-I or HIV-II), thyroid stimulating hormone, apolipoproteins, human chorionic gonadotropin, leutinizing hormone, carcinoembryonic antigen, hepatitis antigen, herpes viruses and other biological and chemical compounds.

The reagent can be used in competitive binding immunoassays. Either bound (that is, complexed) or unbound (that is, uncomplexed) labeled materials can be determined. Physical separation of bound and unbound materials, if desired, can be carried out using any suitable separation technique. In using the analytical elements described below, either vertical or horizontal separation can be used.

In another embodiment, the reagent can be used in what are known in the art as immunometric assays, for example, "sandwich" assays. The details of such assays are provided in U.S. Patent 4,486,530. The reagent of the present invention is useful in such assays where the ligand to be determined has two or more epitopic sites for immunological reaction with two or more receptor molecules. The receptor molecules can be the same or different. The receptors are capable of immunologically reacting with the ligand at different sites. The result of the method is the formation of a complex of the two distinct receptors with the ligand. At least one of the receptors is covalently attached to biotin or avidin (preferably biotin). The corresponding avidin or biotin molecule is attached to the particles described herein. The complex is insolubilized when the avidin and biotin react, and the resulting insolubilized complex can be separated from unreacted material. The particles can be suitably labeled for detection, or one or more of the receptor molecules can be suitably labeled, such as with an enzyme. In a preferred immunometric assay, both receptors are distinct antibodies directed against an antigen, one of which antibodies is enzyme-labeled. They can be the same or different antibodies, whole or fragments, monoclonal or polyclonal.

The method of this invention can be used in either solution or dry assays. By solution assay is meant that the reagents are used in liquid suspension. In dry assays, the reagent is incorporated in a dry analytical element. The simplest element can be composed of an absorbent carrier material, for example, a thin sheet of a self-supporting absorbent or bibulous material, such as filter paper or strips, which has one or more zones, at least one zone containing the reagent of this invention. Other zones can be used to contain other useful reagents. Such elements are known in the art as test strips, diagnostic elements, dip sticks or diagnostic agents.

Useful absorbent carrier materials are insoluble and maintain their structural integrity when exposed to water or biological fluids such as whole blood or serum. Useful elements can be prepared from paper, porous particulate structures, porous polymeric films, cellulose, glass fibers, woven and nonwoven fabrics (synthetic and nonsynthetic). Useful materials and procedures for making such elements are well known in the art.

Preferably, the absorbent carrier material of the dry analytical element of this invention is a porous spreading zone. This zone can be self-supporting (that is, composed of a material rigid enough to maintain its integrity), but preferably it is carried on a separate support. Such a support can be any suitable dimensionally stable, and preferably. nonporous and transparent (that is, radiation transmissive) material which transmits electromagnetic radiation of a wavelength between 200 and 900 nm. A support of choice for a particular element should be compatible with the intended mode of detection (fluorescence, transmission or reflectance spectroscopy). Useful supports can be prepared from paper, metal foils, polystyrene, polyesters, polycarbonates or cellulose esters.

The porous spreading zone can be prepared from any suitable fibrous or non-fibrous material or mixtures of either or both. The void volume and average pore size of this zone can be varied depending upon the use intended. Useful spreading zones can be prepared using materials and procedures described, for example, in U. S. Patents 4,292,272, 3,992,158, 4,258,001 and 4,430,436 and Japanese Patent Publication 57(1982)-101760.

The elements can have two or more discrete zones, either in the same layer or superimposed. At least one of the zones is preferably a porous spreading zone. The other zones can be reagent zones or registration zones as those zones are known in the art, additional spreading zones, radiation-blocking or

filter zones, subbing zones or barrier zones. Preferably, each zone is a separately coated layer, although two or more zones can be separate areas in a single layer of the element.

Preferably, the reagent of this invention is used to detect a multivalent ligand such as human chorionic gonadotropin (hCG), the presence of which in a woman's urine can be an early indicator of pregnancy. An assay for hCG is demonstrated in Example 1 below. This embodiment relating to hCG is presented for illustrative purposes, but it will be understood that the scope of this invention is not so limited. A biological sample (usually urine) suspected of containing the hormone is collected from a patient and contacted with the reagent of this invention. If the ligand is present, a reaction product is formed between the ligand and one or more receptor molecules therefor. One of the receptor molecules can be conjugated to either avidin or biotin which reacts with the reagent of this invention. For example, the reagent can have avidin molecules attached to the particles, which molecules react with biotin molecules which are conjugated with a receptor molecule (such as an antibody) for hCG. The amount of ligand is thereby determined by measuring the presence or amount of the resulting reaction product which is immobilized with the particles. Generally such detection is carried out by measuring the amount of detectable tracer in the complexed or uncomplexed materials.

The method of this invention can also be carried out in a disposable test device wherein a filtration membrane is used to separate complexed materials from uncomplexed materials. Such a device can have one or more wells into which a test sample containing a ligand is added for reaction with the appropriate reagents including the reagent of this invention.

The following examples illustrate the practice of the present invention.

Example 1: Preparation of a Reagent

This example illustrates the preparation of a polymer latex and the attachment of avidin in the Preparation of a reagent of the present invention.

Preparation of Polymer Latex

The three solutions outlined below were continuously added to a 1365 ml vessel containing deoxygenated water at 80° C at the indicated rates:

Solution 1: Styrene (739 g), m & p-(2-chloroethylsulfonylmethyl)styrene (82 g) and 1-dodecanethiol (8.2 g) at 2.5 g/min. for 380 minutes.

Solution 2: Ammonium Persulfate (19.7 g) and distilled, deoxygenated water (1152 g) at 2.14 g/min. for 380 minutes.

Solution 3: Sodium pyrosulfite (9.9 g) and distilled water (1152 g) at 2.27 g/min. for 380 minutes.

After 380 minutes, the reaction was stopped, yielding about 1218 g of latex at 33.4% solids. The latex was dialyzed for 3 days to yield a latex having 27.3% solids and a pH of 5. This latex was diluted to 13.5% solids. NMR analysis confirmed a 96:4 molar ratio of styrene to the second monomer. The resulting latex particles had an average diameter of about 0.67 µm as measured by transmission electron microscopy.

Covalent Attachment of Avidin

A sample (0.75 ml) of the latex described above was diluted to 20 ml with borate buffer (50 mmolar, pH 8.5) and avidin (5 mg) was subsequently added. The resulting suspension was agitated in an end-over-end fashion at 37° C for 18 hours, followed by centrifugation. The supernatant was discarded and the particles washed once with buffer by centrifugation and resuspended in 10 ml borate buffer. Biotin binding analysis (that is, titration with tritium labeled biotin) indicated that avidin had been covalently attached to the particles ($7 \times 10^{-6}$ molar binding sites per 0.3% bead suspension) to form a reagent of the present invention.

Example 2: Preparation of a Larger Sized Reagent

A reagent was prepared similar to that prepared in Example 1 except the polymeric particles in the latex had an average diameter of about 2.5 µm. A sample (0.65 ml at 15.5% solids) of purified latex was diluted to 10 ml with borate buffer (50 mmolar, pH 8.5) and avidin (1 mg) was added. The resulting suspension was agitated end-over-end at room temperature for 24 hours. Radiolabeled tracer analysis (avidin labeled with $^{125}I$) indicated 84% of avidin was covalently bound to the particles following washing of the beads three times.

Example 3: Use of Reagent in the Determination of Human Chorionic Gonadotropin

This example demonstrates the use of the reagent of this invention in an assay to determine hCG.

A test device was used to determine hCG in a urine sample in the following manner. This device contained a filter membrane consisting of a commercially available nylon membrane coated with a protein. A conjugate (3 µg) of biotin and a monoclonal antibody directed to hCG was incorporated into the sample well of the test device. A buffer, 3-(N-morpholino)propanesulfonic acid (2 mg, pH 7.2), was incorporated in a different location in the sample well.

A urine sample, prefiltered to remove impurities, and containing about 50 mI.U./ml of hCG, was added to the sample well followed by addition of a second monoclonal antibody to hCG which is labeled with horseradish peroxidase (40 µl of a $10^{-9}$ molar solution). A complex of antigen (hCG) and the two antibodies was formed in solution during a one minute incubation period at room temperature.

A sample of the immunoreactive reagent of this invention was then added to the well containing the complex. This sample contained 40 µl of a 0.42% dispersion of poly[styrene-co-m & p-(2-chloroethylsulfonylmethyl)styrene] particles to which avidin was attached. After this addition, fluid in the well was allowed to drain through the membrane of the test device, and a wash solution containing 0.1 molar sodium phosphate (200 µl) and sodium dodecylsulfate(10 mmolar) was added. A dye-providing composition (40 µl) was then added. This composition comprised 2-(4-hydroxy-3,5-dimethoxyphenyl)-4,5-bis(4-methoxyphenyl)-imidazoleleuco dye (0.005% in 40 µl solution), diethylenetriaminepentaacetic acid (10 µmolar) chelating agent, 4′-hydroxyacetanilide (5 mmolar) electron transfer agent, hydrogen peroxide (10 mmolar) in an aqueous solution buffered to pH 7 with sodium phosphate with a minor amount of methanol. After two minutes of reaction, a detectable dye was formed in the complex retained in the sample well on the membrane. The amount of dye was measured by converting the measured reflectance to transmittance ($D_T$) using conventional equipment and the Williams-Clapper transform (J. Opt. Soc. Am. , 43, p.595, 1953). The amount of measured dye was an indication of the presence of hCG in the urine sample tested.

Example 4: Preparation of Reagent Using Particles Having Reactive Chloromethyl Groups

Poly(styrene-co-m & p-chloromethylstyrene) (77.3:22.7 molar ratio) particles having an average particle diameter size of 2.9 µm were prepared using a continuous emulsion polymerization procedure similar to that described in Example 1 above. A sample of the resulting latex (0.82 ml, 12.2% solids) was diluted with 20 ml of 0.05 molar borate buffer (pH 8.5, containing 0.01% thiomersal germicide). Avidin (5 mg) was added to the diluted latex and the resulting suspension was rotated end-over-end at 37° C for 24 hours. The avidin was thereby attached to the particles through the chloromethyl groups on the particle surfaces. Biotin binding capacity, as measured by the procedure described in Example 1, was 1.5 x $10^{-6}$ molar sites per 0.3% of diluted particle suspension.

Example 5: Preparation of Reagent Having Biotin Attached to the Particles and Use in an Assay for hCG

This example demonstrates the preparation of an immunoreactive reagent of this invention whereby biotin is attached to water insoluble polymeric particles, and the use of the reagent in an assay for hCG.

Preparation of Reagent

A solution of borate buffer (50 ml, 0.05 molar, pH 8.5) containing 0.01% thiomersal germicide was placed in a centrifuge tube, followed by the addition of a solution (6 ml) of casein (1 mg/ml) in deionized distilled water. The tube was capped and shaken vigorously, then an aqueous dispersion (1.23 ml of 12.22% solids) of poly(styrene-co-m & p-chloromethylstyrene)(77.3:22.7 molar ratio) polymeric particles (average size about 2.7 µm) was added, and the tube was capped and rotated end-over-end for 24 hours at 37° C. The resulting particles had casein attached to the outer surfaces through the reactive chloromethyl groups.

The casein-particle reagent was then washed with glycine buffer (0.1 molar, pH 8.5) containing 0.01% thiomersal, and then resuspended in glycine buffer (0.1 molar) containing 0.01% thiomersal to produce a dispersion (0.3% solids) of a casein-particle reagent.

A sample of the dispersion just described (100 ml) was centrifuged, washed with sodium bicarbonate solution (80 ml, 100 mmolar), centrifuged again at about 15,000 rpm for 5 to 6 minutes, and resuspended in sodium bicarbonate (100 mmolar) to produce a 100 ml bead suspension. This suspension was treated with a mixture of biotin esterified with N-hydroxysuccinimide (10 mg), and allowed to incubate for about 90

minutes. The resulting product was centrifuged, washed with sodium phosphate buffer (200 ml, 50 mmolar, pH 7.2) containing diethylenetriaminepentaacetic acid (5 µmolar), centrifuged again and resuspended in sodium phosphate (100 ml, 50 mmolar, pH 7.2, 0.3% solids).

Assay for hCG

A three-well test device like that described above, was pretreated by contacting the nylon microporous filter in two of the wells with a solution of a protein (2% solids). The membrane in the third test well was not so treated.

A stock solution of hCG (GC-10 chorionic gonadotropin) was prepared with phosphate buffered saline solution (50 mmolar sodium phosphate, 150 mmolar sodium chloride, pH 7) to comprise 5000 mI.U. hCG/ml for use as a test solution.

A sample (150 µl) of this test solution was placed in each of the two casein-treated test wells of the test device, and the following reagents were immediately placed in each of the three wells:

a) 40 µl of the biotinylated reagent described above, diluted 1:10.

b) 40 µl of an avidin-anti-hCG conjugate diluted 1:200. This conjugate was prepared from a purified monoclonal antibody against β-hCG (Immunoresearch) coupled to thiolated avidin by the thiol/maleimide procedure of Chen et al, Clin. Chem., 30(9), pp. 1446-1451 (1984) having 0.01% thiomersal added. The solution was characterized as having an optical density of 0.072 at 280 nm before dilution.

c) 40 µl of a peroxidase labeled monoclonal antibody against hCG diluted 1:250. This conjugate was prepared by coupling the antibody to horseradish peroxidase essentially as described by Chen et al, supra. The solution comprised 0.49 mg/ml of antibody before dilution.

The mixture in each test well was allowed to incubate for 30 seconds, then fluid was drained through the membrane, and each well was washed with 8 drops of a solution of sodium phosphate (100 mmolar) and sodium dodecylsulfate (10 mmolar). To each well was then added one drop of a dye composition similar to that described above.

After incubation for 15 seconds, the fluid was drained through the membrane in each test well, and the color formed on the membrane was observed after another three minutes. The color formed was indicative of the presence of hCG in the test solution.

The assay described above was repeated twice except that the fluid was drained in each test well after 15 minutes incubation, followed by color observation after one or two minutes. Also, in the third assay, the biotinylated reagent dispersion was used undiluted. The following table shows the results from these assays. These data show that a positive color is generated in the test wells containing the hCG test solution. However, in the third well into which no hCG was added, there was essentially no color formation.

| Assay | Test Well | Casein Pretreatment | Incubation Time | Color Observed | | |
|---|---|---|---|---|---|---|
| | | | | 1 Min. | 2 Min. | 3 Min. |
| 1* | 1 | Yes | 30 sec. | -- | -- | Light Pink |
| 1* | 2 | Yes | 30 sec. | -- | -- | Light Pink |
| 1* | 3 | No | 30 sec. | -- | -- | White with Pink Edges |
| 2* | 1 | Yes | 15 min. | Medium Pink | Medium Pink (Mottled) | -- |
| 2* | 2 | Yes | 15 min. | Medium Pink | Medium Pink (Mottled) | -- |
| 2* | 3 | No | 15 min. | White | White with some Pink | -- |
| 3** | 1 | Yes | 15 min. | -- | Very Pale Pink | -- |
| 3** | 2 | Yes | 15 min. | -- | Very Pale Pink | -- |
| 3** | 3 | No | 15 min. | -- | White | -- |

*Biotinylated Reagent Dispersion Diluted 1:10
**Biotinylated Reagent Dispersion Undiluted

Example 6: Use of Reagent to Determine Luteinizing Hormone (LH)

Immobilization of Avidin on Particles

A solution (50 ml, 0.05 molar, pH 8.5) of borate buffer containing thiomersal germicide (0.01%) was

placed in a polypropylene centrifuge tube, and to it was added 6 ml of a solution of egg white avidin (6 mg) dissolved in 6 ml of deionized distilled water. The tube was then capped and shaken vigorously, followed by addition of 1.35 ml of a dispersion (15.5% solids) of poly[styrene-co-m & p-(2-chloroethylsulfonylmethyl)-styrene] (95.5:4.5 molar ratio) beads (average size of 2.54 µmeters) and rotation end-over-end for 24 hours.

The polymer beads having avidin covalently attached thereto were washed with glycine buffer (0.1 molar, pH 8.5) containing 0.01% thiomersal, and then resuspended in glycine buffer (0.1 molar) containing 0.01% thiomersal to produce a dispersion containing an insoluble separation specific binding reagent (0.3% solids).

Assay for LH

A test device having three test wells like that described above, having a 5 µm mesh nylon filter membrane which had been pretreated with casein in each well, was used in this assay. Also used in the assay were: a biotinylated antibody to LH (0.044 mg/ml in phosphate buffered saline solution) prepared similarly to the biotinylated antibody to hCG described above, a horseradish peroxidase labeled antibody to LH (0.0015 mg/ml in phosphate buffered saline solution containing 0.5% bovine serum albumin), and the reagent described above comprising avidin on beads (concentrated to 0.9% solids, pH 8.5).

Several urine samples were tested in this assay:

(a) a sample collected the 13 th day of a woman's menstrual cycle containing 18 mI.U. LH/ml, and

(b) a sample collected the 14th day of the woman's menstrual cycle containing 64 mI.U. LH/ml.

Both of these samples were taken from the same person and the LH content was determined by an LH radioimmunoassay kit available from Diagnostic Products Corporation.

A mixture of urine sample (a) (200 ml), the peroxidase labeled antibody (35 µl) and the biotinylated antibody (10 µl) was prepared and incubated at room temperature for two minutes. The insolubilized avidin reagent (40 µl) was then added to the mixture and incubation was continued for another five minutes. The mixture was then transferred to one of the test wells of the test device, and fluid and unreacted materials were drained away leaving insolubilized complex formed during incubation on the filter membrane. The remaining insolubilized product was then washed twice with phosphate buffered saline solution (125 µl) containing 0.1% TWEEN 20 (trade mark) surfactant (a sorbitan monolaurate nonionic surfactant), filtered again and then contacted with a dye-providing solution (50 µl, pH 7) like that described above.

Urine sample (b) was similarly assayed using a second test well of the test device.

In both test wells, a color was seen on the filter membrane within five minutes. The color in the first well was light pink in color whereas the color in the second well was bright red in color.

Example 7: Assay for Streptococcus A Antigen

This example demonstrates the preparation and use of a reagent of this invention to determine Streptococcus A antigen in a fluid sample.

The reagent was prepared by attaching avidin to poly(styrene-co-m & p chloromethylstyrene-co-2-hydroxyethyl acrylate) (69:30:1 molar ratio) particles (0.69 µm average size) using a procedure similar to that described in Example 1. The particles are also prepared using an emulsion polymerization procedure like that described in Example 1.

A sample (0.15% solids) of the reagent described above was mixed with a biotinylated antibody to Streptococcus A antigen (20% antibody w/w) and a fluid sample containing extracted Streptococcus A antigen, and the resulting mixture was incubated for 1 hour. After that time, it was observed that an agglutination reaction had occurred indicating that the antibody had reacted with the antigen, and the resulting complex was insolubilized by the reaction of avidin with biotin.

**Claims**

**1.** A water-insoluble reagent comprising:

a polymeric particle comprising a polymer on at least its outer surface which is derived from one or more ethylenically unsaturated polymerizable monomers,

the reagent characterized in that at least one of the monomers has reactive groups which are activated 2-substituted ethylsulfonyl, vinylsulfonyl or active halogen atom groups, and the particle is covalently attached through the reactive groups on the outer surface of the particle to avidin, biotin or an avidin or biotin derivative.

2. The reagent as claimed in claim 1 having a detectable tracer material associated therewith.

3. The reagent as claimed in either claim 1 or 2 having avidin or a derivative thereof attached to the particle.

4. The reagent as claimed in any one of claims 1 to 3 wherein the polymer is represented by the formula:

$$-(A)_x-(B)_y-(D)_z-$$

wherein -A- represents recurring units derived from one or more hydrophobic ethylenically unsaturated polymerizable monomers,

-B- represents recurring units derived from one or more ethylenically unsaturated polymerizable monomers having a reactive group which is an activated 2-substituted ethylsulfonyl, vinylsulfonyl or active halogen atom,

-D- represents recurring units derived from one or more ethylenically unsaturated polymerizable monomers other than those represented by -A- or -B-,

x is from 0 to 99.9 mole percent, y is from 0.1 to 100 mole percent, and z is from 0 to 20 mole percent.

5. The reagent as claimed in any one of claims 1 to 4 wherein the activated 2-substituted ethylsulfonyl and vinylsulfonyl monomers are represented by the formula:

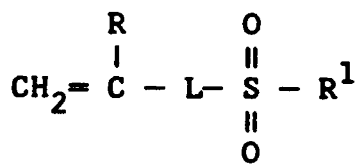

wherein R is hydrogen or substituted or unsubstituted alkyl,

$R^1$ is $-CH=CHR^2$ or $-CH_2CH_2X$ wherein X is a leaving group which is displaced by a nucleophile or is eliminated in the form of HX by treatment with a base, and $R^2$ is hydrogen, substituted or unsubstituted alkyl, or substituted or unsubstituted aryl, and

L is a substituted or unsubstituted alkylene, substituted or unsubstituted arylene, a combination of one or more of each of the alkylene and arylene groups, or such combination interrupted or terminated with one or more amide or ester groups.

6. The reagent as claimed in claim 5 wherein R is hydrogen or methyl, $R^1$ is $-CH_2CH_2X$ and L is substituted or unsubstituted phenylenealkylene, carbonyliminoarylenealkylene, or carbonyliminoalkyleneiminocarbonylalkylene.

7. An element comprising an absorbent carrier material having one or more zones, the element characterized in that it contains in one or more of the zones a water-insoluble reagent as claimed in any one of claims 1 to 6.

8. A method for the determination of a compound of biological interest in an aqueous liquid comprising:

A. contacting the liquid with a water-insoluble reagent as claimed in any one of claims 1 to 6,

B. forming a reaction product of the reagent with the biological compound, and

C. determining the amount of biological compound as a result of the presence of the reaction product.

9. The method as claimed in claim 8 for the determination of hCG wherein the immunoreactive species of the reagent is a first antibody against hCG, and wherein the method is carried out with a second antibody against hCG which is labeled for detection.

10. The method as claimed in claim 9 wherein the second antibody is labeled with an enzyme.

**11.** The method as claimed in claim 8 for the determination of a human retrovirus.

**12.** A method for the determination of a compound of biological interest in an aqueous liquid comprising:
A. contacting the liquid with a water-insoluble reagent comprising:
a core/shell polymeric particle in which the shell comprises a polymer which is represented by the formula:

$$-(A)_x-(B)_y-(D)_z-$$

wherein -A- represents recurring units derived from one or more hydrophobic ethylenically unsaturated monomers,
-B- represents recurring units derived from one or more ethylenically unsaturated monomers having a reactive group which is an activated 2-substituted ethylsulfonyl, vinylsulfonyl or active halogen atom,
-D- represents recurring units derived from one or more ethylenically unsaturated monomers other than those represented by -A- or -B-,
x is from 0 to 99.9 mole percent, y is from 0.1 to 100 mole percent, and z is from 0 to 20 mole percent,
the particle being covalently attached through the reactive groups on the outer surface of the shell to avidin or a derivative thereof,
B. forming a reaction product of the reagent with a biotinylated reagent comprised of biotin or a derivative thereof attached to a first receptor molecule for the biological compound so as to form an insoluble specific binding complex between avidin and the biotinylated reagent,
C. prior to, simultaneously with or subsequent to contacting step A, contacting the biological compound with a second receptor molecule which is capable of participating in a specific binding reaction with the biological compound but which is not reactive with the first receptor molecule, the second receptor being labeled with a detectable tracer material,
so as to form a labeled insoluble complex, and
D. detecting the labeled insoluble complex.

**13.** The method as claimed in claim 12 wherein the biological compound is an antigen and the first and second receptors are distinct antibodies to the antigen.

**Revendications**

**1.** Réactif insoluble dans l'eau comprenant :
une particule polymère comprenant un polymère sur au moins sa surface externe, qui est formé à partir d'un ou plusieurs monomères polymérisables à insaturation éthylénique,
réactif caractérisé en ce qu'au moins un des monomères a des groupes réactifs qui sont des groupes éthylsulfonyle activé substitué en position 2, vinylsulfonyle ou comprenant un atome d'halogène actif, et la particule est rattachée par une liaison covalente par les groupes réactifs sur la surface externe de la particule à de l'avidine, de la biotine ou des dérivés d'avidine ou de biotine.

**2.** Réactif selon la revendication 1 auquel est associée une substance indicatrice détectable.

**3.** Réactif selon la revendication 1 ou 2 dans lequel de l'avidine ou un dérivé d'avidine est rattaché à la particule.

**4.** Réactif selon l'une quelconque des revendications 1 à 3 dans lequel le polymère est représenté par la formule :

$$-(A)_x-(B)_y-(D)_z-$$

où -A- représente des motifs dérivés d'un ou plusieurs monomères polymérisables hydrophobes à

insaturation éthylénique,
- B- représente des motifs dérivés d'un ou plusieurs monomères polymérisables à insaturation éthylénique ayant un groupe réactif qui est un groupe éthylsulfonyle activé substitué en position 2 ou vinylsulfonyle ou un groupe comprenant un atome d'halogène actif,
- D - représente des motifs dérivés d'un ou plusieurs monomères à insaturation éthylénique autres que ceux représentés par - A - ou - B -,
x est de 0 à 99,9 moles %, y est de 0,1 à 100 mole % et z est de 0 à 20 mole %.

**5.** Réactif selon l'une quelconque des revendications 1 à 4 dans lequel les monomères éthylsulfonyle activé substitué en position 2 et vinylsulfonyle sont représentés par la formule :

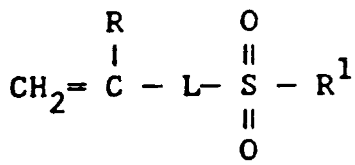

où R est un hydrogène ou un alkyle substitué ou non,
$R^1$ est $-CH=CHR^2$ ou $-CH_2CH_2X$ où X est un groupe de clivage qui est déplacé par un nucléophile ou est éliminé sous la forme HX par traitement avec une base, et $R^2$ est un hydrogène, un alkyle substitué ou non, ou un aryle substitué ou non, et
L est un alkylène substitué ou non, un arylène substitué ou non, une combinaison d'un ou plusieurs groupes alkylène et arylène ou une telle combinaison interrompue ou terminée par un ou plusieurs groupes amide ou esters.

**6.** Réactif selon la revendication 5 dans lequel R est un hydrogène ou un groupe méthyle, $R^1$ est $-CH_2CH_2X$ et L est un phénylènealkylène, carbonyliminoalkylène ou carbonyliminoalkylèneiminocarbonylalkylène.

**7.** Elément comprenant une substance porteuse absorbante ayant une ou plusieurs zones, élément caractérisé en ce qu'il contient une ou plusieurs zones d'un réactif insoluble dans l'eau selon l'une quelconque des revendications 1 à 6.

**8.** Méthode pour le dosage d'un composé présentant un intérêt du point de vue biologique dans un liquide aqueux comprenant :
A. la mise en contact du liquide avec un réactif insoluble selon l'une quelconque des revendications 1 à 6,
B. la formation d'un produit de réaction avec le composé biologique, et
C. le dosage de la quantité de composé biologique en rapport avec la présence du produit de réaction.

**9.** Méthode selon la revendication 8 pour doser l'hCG dans laquelle l'espèce immunoréactive du réactif est un premier anticorps anti-hCG, et dans laquelle la méthode est réalisée avec un second anticorps anti-hCG qui est marqué pour pouvoir être détecté.

**10.** Méthode selon la revendication 9 dans laquelle le second anticorps est marqué avec un enzyme.

**11.** Méthode selon la revendication 8 pour le dosage d'un rétrovirus humain.

**12.** Méthode pour le dosage d'un composé présentant un intérêt du point de vue biologique dans un liquide aqueux comprenant
A. la mise en contact du liquide avec un réactif insoluble dans l'eau comprenant :
une particule polymère du type noyau/enveloppe (core/shell) dans laquelle l'enveloppe comprend un polymère représenté par la formule :

$$-(A)_x-(B)_y-(D)_z-$$

où -A- représente des motifs dérivés d'un ou plusieurs monomères polymérisables hydrophobes à insaturation éthylénique,
- B- représente des motifs dérivés d'un ou plusieurs monomères polymérisables à insaturation éthylénique ayant un groupe réactif qui est un groupe éthylsulfonyle activé substitué en position 2 ou vinylsulfonyle ou un groupe comprenant un atome d'halogène actif,
- D - représente des motifs dérivés d'un ou plusieurs monomères à insaturation éthylénique autres que ceux représentés par - A - ou - B -,
x est de 0 à 99,9 moles %, y est de 0,1 à 100 mole % et z est de 0 à 20 mole %,
la particule étant rattachée par une liaison covalente par les groupes réactifs sur la surface externe de la particule à de l'avidine, ou à un dérivé de l'avidine,
B. la formation d'un produit de réaction du réactif avec un réactif biotinylé composé de biotine ou d'un de ses dérivés rattaché à une première molécule réceptrice du composé biologique pour former entre l'avidine et le réactif biotinylé un complexe liant spécifique insoluble,
C. avant, pendant ou après l'étape de mise en contact A, la mise en contact du composé biologique avec une deuxième molécule réceptrice capable de participer à une réaction de liaison avec le composé biologique mais ne réagissant pas avec la première molécule réceptrice, le second récepteur étant marqué avec une substance marquante détectable,
pour former un complexe insoluble marqué, et
D. doser le complexe insoluble marqué.

**13.** Méthode selon la revendication 12 dans laquelle le composé biologique est un antigène et le premier et second récepteurs sont des anticorps distincts de l'antigène.

**Patentansprüche**

**1.** In Wasser unlösliches Reagens mit: einem polymeren Teilchen mit einem Polymer auf mindestens seiner äußeren Oberfläche, das sich von einem oder mehreren ethylenisch ungesättigten polymerisierbaren Monomeren ableitet,
dadurch gekennzeichnet, daß mindestens eines der Monomeren reaktive Gruppen aufweist, die aus aktivierten 2-substituierten Ethylsulfonyl-, Vinylsulfonyl- oder aktiven Halogenatomgruppen bestehen, wobei das Teilchen durch die reaktiven Gruppen auf der äußeren Oberfläche des Teilchens kovalent an Avidin, Biotin oder ein Avidin- oder Biotinderivat gebunden ist.

**2.** Reagens nach Anspruch 1, das mit einem Erkennungsmaterial assoziiert ist.

**3.** Reagens nach einem der Ansprüche 1 oder 2, bei dem Avidin oder ein Derivat desselben an das Partikel gebunden ist.

**4.** Reagens nach einem der Ansprüche 1 bis 3, in dem das Polymer durch die Formel:

$$-(A)_x-(B)_y-(D)_z-$$

wiedergegeben wird,
in der -A- wiederkehrende Einheiten darstellt, die sich von einem oder mehreren hydrophoben, ethylenisch ungesättigten polymerisierbaren Monomeren ableiten,
-B- wiederkehrende Einheiten darstellt, die sich von einem oder mehreren ethylenisch ungesättigten polymerisierbaren Monomeren ableiten, die eine reaktive Gruppe aufweisen, bei der es sich um eine aktivierte, 2-substituierte Ethylsulfonyl-, Vinylsulfonyl- oder aktive Halogenatomgruppe handelt,
-D- wiederkehrende Einheiten darstellt, die sich von einem oder mehreren ethylenisch ungesättigten polymerisierbaren Monomeren ableiten, die von denen, die durch -A- oder -B-dargestellt werden, verschieden sind,
und in der

x für 0 bis 99,9 Molprozent, y für 0,1 bis 100 Molprozent und z für 0 bis 20 Molprozent stehen.

**5.** Reagens nach einem der Ansprüche 1 bis 4, in dem die aktivierten 2-substituierten Ethylsulfonyl- und Vinylsulfonylmonomeren durch die folgende Formel wiedergegeben werden:

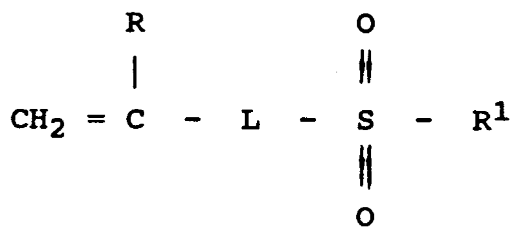

in der bedeuten:

R Wasserstoff oder substituiertes oder unsubstituiertes Alkyl;

$R^1$ gleich $-CH=CHR^2$ oder $-CH_2CH_2X$ ist, worin X für eine entfernbare Gruppe steht, die durch ein Nukleophil verdrängbar ist, oder durch Behandlung mit einer Base in Form von HX eliminierbar ist, und $R^2$ für Wasserstoff, substituiertes oder unsubstituiertes Alkyl, oder substituiertes oder unsubstituiertes Aryl steht, und

L steht für substituiertes oder unsubstituiertes Alkylen, substituiertes oder unsubstituiertes Arylen, eine Kombination aus einer oder mehreren Alkylen- und Arylengruppen oder solchen Kombinationen, die durch eine oder mehrere Amid- oder Estergruppen unterbrochen sind oder abgeschlossen werden.

**6.** Reagens nach Anspruch 5, in dem R für Wasserstoff oder Methyl steht, $R^1$ eine Gruppe der Formel $-CH_2CH_2X$ ist und L für substituiertes oder unsubstituiertes Phenylenalkylen, Carbonyliminoarylenalkylen oder Carbonyliminoalkyleniminocarbonylalkylen steht.

**7.** Element mit einem absorbierenden Trägermaterial mit einer oder mehreren Zonen, dadurch gekennzeichnet, daß es in einer oder mehreren Zonen ein in Wasser unlösliches Reagens nach einem der Ansprüche 1 bis 6 enthält.

**8.** Verfahren zur Bestimmung einer Verbindung von biologischem Interesse in einer wäßrigen Flüssigkeit, bei dem man:

A. die Flüssigkeit mit einem in Wasser unlöslichen Reagens nach einem der Ansprüche 1 bis 6 kontaktiert,

B. ein Reaktionsprodukt aus dem Reagens und der biologischen Verbindung erzeugt, und

C. die Menge an biologischer Verbindung als Ergebnis des Vorhandenseins des Reaktionsproduktes bestimmt.

**9.** Verfahren nach Anspruch 8, für die Bestimmung von hCG, bei der die immunoreaktive Komponente des Reagens ein erster Antikörper für hCG ist, und das Verfahren mit einem zweiten Antikörper für hCG durchgeführt wird, der zur Bestimmung markiert ist.

**10.** Verfahren nach Anspruch 9, in dem der zweite Antikörper mit einem Enzym markiert ist.

**11.** Verfahren nach Anspruch 8, zur Bestimmung eines menschlichen Retrovirus.

**12.** Verfahren zur Bestimmung einer Verbindung von biologischem Interesse in einer wäßrigen Flüssigkeit mit den Stufen:

A. Kontaktieren der Flüssigkeit mit einem in Wasser unlöslichen Reagens mit einem polymeren Kern/Hüllen-Partikel, in dem die Hülle aus einem Polymeren aufgebaut ist, das durch die folgende Formel wiedergegeben wird:

$$-(A)_x-(B)_y-(D)_z$$

in der
-A- wiederkehrende Einheiten darstellt, die sich von einem oder mehreren hydrophoben, ethylenisch ungesättigten Monomeren ableiten,
-B- wiederkehrende Einheiten darstellt, die sich von einem oder mehreren ethylenich ungesättigten Monomeren ableiten, die eine reaktive Gruppe aufweisen, bei der es sich um eine aktivierte 2-substituierte Ethylsulfonyl-, Vinylsulfonyl- oder aktive Halogenatomgruppe handelt,
-D- wiederkehrende Einheiten darstellt, die sich von einem oder mehreren ethylenisch ungesättigten Monomeren ableiten, die von denen, die durch -A- oder -B-dargestellt werden, verschieden sind, und in der x für 0 bis 99,9 Molprozent, y für 0,1 bis 100 Molprozent und z für 0 bis 20 Molprozent stehen,
wobei das Partikel durch die reaktiven Gruppen auf der äußeren Oberfläche der Hülle kovalent an Avidin oder ein Derivat hiervon gebunden ist;
B. Erzeugung eines Reaktionsproduktes aus dem Reagens und einem biotinylierten Reagens aus Biotin oder einem Derivat desselben, das an ein erstes Rezeptormolekül für die biologische Verbindung gebunden ist, unter Erzeugung eines unlöslichen spezifischen Bindungskomplexes zwischen Avidin und dem biotinylierten Reagens,
C. Kontaktieren der biologischen Verbindung mit einem zweiten Rezeptormolekül, das in der Lage ist, an einer spezifischen Bindungsreaktion mit der biologischen Verbindung teilzunehmen, das jedoch mit dem ersten Rezeptormolekül nicht reagiert, wobei der zweite Rezeptor mit einer bestimmbaren Gruppe markiert ist, vor Durchführung, gleichzeitig mit oder nach Durchführung der Kontaktierungsstufe A, unter Bildung eines markierten unlöslichen Komplexes und
D. Bestimmung des markierten unlöslichen Komplexes.

**13.** Verfahren nach Anspruch 12, in dem die biologische Verbindung ein Antigen ist und in dem die ersten und zweiten Rezeptoren verschiedene Antikörper für das Antigen sind.